# EUROPEAN PATENT SPECIFICATION

(11) **EP 2 926 737 B1**
(45) Date of publication and mention of the grant of the patent: **30.10.2019**
(21) Application number: 14185715.1
(22) Date of filing: 22.09.2014
(51) Int. Cl.: A61B 8/08, A61B 8/13

(54) **Ultrasound diagnostic apparatus and method of operating the same**
Ultraschalldiagnosevorrichtung und Verfahren zum Betrieb davon
Appareil de diagnostic par ultrasons et son procédé de fonctionnement

(30) Priority: 01.04.2014 KR 20140038750
(43) Date of publication of application: 07.10.2015
(73) Proprietor: Samsung Medison Co., Ltd., Gangwon-do 250-870 (KR)
(72) Inventor: Kim, Nam-woong, Hongcheon-gun Gangwon-do (KR); Jin, Gil-ju, Hongcheon-gun Gangwon-do (KR)
(74) Representative: Frey, Sven Holger

(56) References cited:
- WO-A2-03/047433
- AU-A4- 2011 100 208
- JP-A- 2000 102 540
- US-A- 5 538 004
- US-A1- 2014 031 688

## Description

### BACKGROUND

### 1. Field

One or more embodiments of the present invention relate to an ultrasound diagnostic apparatus and a method of operating the same, and more particularly, to an ultrasound diagnostic apparatus and a method of operating the same to correct shaking of an ultrasound image caused by hand shaking.

### 2. Description of the Related Art

Ultrasound diagnostic apparatuses irradiate an ultrasound signal, generated from a transducer of a probe, onto an object and receive information of an echo signal reflected from the object, thereby obtaining an image of an internal part of the object. In particular, ultrasound diagnostic apparatuses are used for the medical purpose of observing the inside of an object, detecting a foreign material, and assessing an injury. Ultrasound diagnostic apparatuses have stabilities higher than those of diagnostic apparatuses using X-rays, display an image in real time, and are safe because there is no exposure to radioactivity, and thus may be widely used along with other image diagnostic apparatuses.

Ultrasound diagnostic apparatuses may provide a brightness (B) mode in which a reflection coefficient of an ultrasound signal reflected from an object is shown as a two-dimensional (2D) image, a Doppler mode image in which an image of a moving object (particularly, blood flow) is shown by using the Doppler effect, and an elastic mode image in which a reaction difference between when compression is applied to an object and when compression is not applied to the object is expressed as an image.

WO 03/047433 A2 discloses an ultrasonic diagnostic imaging system that comprises a "zoom" feature and is configured to stabilize anatomical images in the presence of probe motion, such as slow undesired motion of the probe due to an uncooperative patient or organ motion.

### SUMMARY

The invention is defined in the claims. The present invention provides an ultrasound diagnostic apparatus and a method of operating the same to correct shaking of a displayed ultrasound image caused by an undesired motion of a probe such as shaking of a user's hand.

Additional aspects will be set forth in part in the description which follows and, in part, will be apparent from the description, or may be learned by practice of the presented embodiments.

According to one or more embodiments of the present invention, a method of operating an ultrasound diagnostic apparatus is provided as defined in independent claim 1.

The detecting of the shaking may include detecting a motion of at least one reference pixel included in the ROI among a plurality of pixels included in the candidate image.

The detecting of the shaking may include calculating a motion vector indicating the motion of the at least one reference pixel, and the displaying of the some images may include moving the set ROI in the same direction as a direction of the motion vector, and displaying the portion of the candidate image corresponding to the moved ROI among the plurality of candidate images.

The detecting of the shaking may include calculating a motion of the probe to determine whether the motion of the probe corresponds to a certain frequency band.

The detecting of the shaking may include sensing the shaking of the probe by using a sensor.

The displaying of the portion of the candidate image may include moving the set ROI in a direction opposite to the sensing value to display some images corresponding to the moved ROI among the plurality of candidate images.

The method may further include displaying the detected shaking of the probe.

According to one or more embodiments of the present invention, an ultrasound diagnostic apparatus is provided as defined in independent claim 8.

The shaking detecting unit may detect a motion of at least one reference pixel included in the ROI among a plurality of pixels included in the candidate image.

The shaking detecting unit may calculate a motion vector indicating the motion of the at least one reference pixel, and the display unit may move the set ROI in the same direction as a direction of the motion vector, and display the portion of the candidate image corresponding to the moved ROI among the plurality of candidate images.

The shaking detecting unit may calculate a motion of the probe to determine whether the motion of the probe corresponds to a certain frequency band.

The shaking detecting unit may sense the shaking of the probe by using a sensor.

The display unit may move the set ROI in a direction opposite to the sensing value to display the portion of the candidate image corresponding to the moved ROI among the plurality of candidate images.

The display unit may display the detected shaking of the probe.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram illustrating a configuration of an ultrasound diagnostic apparatus according to an embodiment of the present invention;
FIG. 2 is a block diagram illustrating a configuration of an ultrasound diagnostic apparatus according to an embodiment of the present invention;
FIG. 3 is a flowchart illustrating a method of operating an ultrasound diagnostic apparatus, according to an embodiment of the present invention; and
FIGS. 4 to 8 are diagrams referred to for describing an operating method of FIG. 3.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

All terms including descriptive or technical terms which are used herein should be construed as having meanings that are obvious to one of ordinary skill in the art. However, some terms may be arbitrarily selected by the applicant, and in this case, the meaning of the selected terms will be described in detail in the detailed description of the invention. Thus, the terms used herein have to be defined based on the meaning of the terms together with the description throughout the specification.

Also, when a part "includes" or "comprises" an element, unless there is a particular description contrary thereto, the part may further include other elements, not excluding the other elements. Moreover, each of terms such as "...unit" and "module" described in specification denotes an element for performing at least one function or operation, and may be implemented in hardware, software or a combination of hardware and software.

The term "ultrasound image" used herein denotes an image of an object acquired by using an ultrasound wave. Also, the term "object" used herein may include a person, an animal, a part of the person, or a part of the animal. For example, an object may include an organ such as a liver, a heart, a womb, a brain, breasts, an abdomen, or the like, or a blood vessel. Also, the term "object" may include a phantom. The phantom denotes a material having a volume, density, and effective atomic number similar to those of an organism, and may include a spherical phantom having a characteristic similar to a physical body.

Moreover, the ultrasound image may be of various types. For example, the ultrasound image may be at least one of an amplitude (A) mode image, a brightness (B) mode image, a color (C) mode image, and a Doppler (D) mode image. Also, according to an embodiment of the present invention, the ultrasound image may be a two-dimensional (2D) image or a three-dimensional (3D) image.

Moreover, the term "user" used herein is a medical expert, and may be a medical doctor, a nurse, a medical technologist, a medical image expert, or the like, or may be an engineer who repairs a medical apparatus. However, the user is not limited thereto.

The present invention will now be described more fully with reference to the accompanying drawings, in which exemplary embodiments of the invention are shown. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art. The invention is defined in the claims. In the following description, well-known functions or constructions are not described in detail since they would obscure the invention with unnecessary detail. Throughout the specification, like reference numerals in the drawings denote like elements.

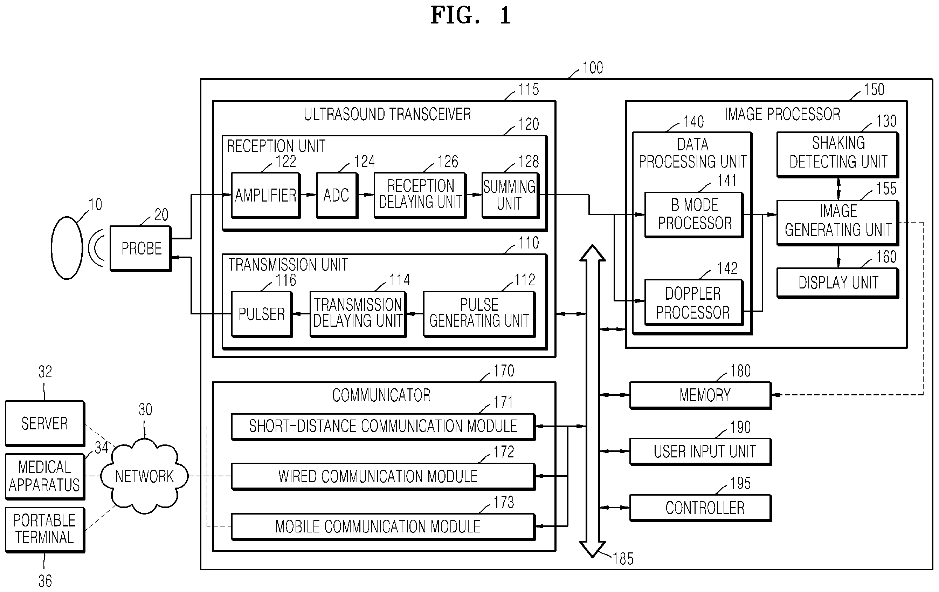

FIG. 1 is a block diagram illustrating a configuration of an ultrasound diagnostic apparatus 100 according to an embodiment of the present invention. Referring to FIG. 1, the ultrasound diagnostic apparatus 100 according to an embodiment of the present invention includes a probe 20, an ultrasound transceiver 115, an image processor 150, a communicator 170, a memory 180, a user input unit 190, and a controller 195. The above-described elements may be connected to each other through a bus 185. Also, the image processor 150 may include a data processing unit 140, a shaking detecting unit 130, an image generating unit 155, and a display unit 160.

The probe 20 transmits ultrasound waves to an object 10 based on a driving signal applied by the ultrasound transceiver 115 and receives echo signals reflected by the object 10. The probe 20 includes a plurality of transducers, and the plurality of transducers oscillate based on electric signals transmitted thereto and generate acoustic energy, that is, ultrasound waves. Furthermore, the probe 20 may be connected to the main body of the ultrasound diagnostic apparatus 100 in a wire or wireless manner. According to embodiments of the present invention, the ultrasound diagnostic apparatus 100 may include a plurality of probes 20.

A transmission unit 110 supplies a driving signal to the probe 20 and includes a pulse generating unit 112, a transmission delaying unit 114, and a pulser 116. The pulse generating unit 112 generates pulses for forming ultrasound waves based on a predetermined pulse repetition frequency (PRF), and the transmission delaying unit 114 applies a delay time to the pulses for determining a transmission directionality. Pulses to which a delay time is applied correspond to a plurality of piezoelectric vibrators included in the probe 20, respectively. The pulser 116 applies a driving signal (or a driving pulse) to the probe 20 as a timing corresponding to each pulse to which a delay time is applied.

A reception unit 120 generates ultrasound data by processing echo signals received from the probe 20 and may include an amplifier 122, an analog-digital converter (ADC) 124, a reception delaying unit 126, and a summing unit 128. The amplifier 122 amplifies echo signals in each channel, and the ADC 124 analog-to-digital converts the amplified echo signals. The reception delaying unit 126 applies delay times for determining reception directionality to the digital-converted echo signals, and the summing unit 128 generates ultrasound data by summing the echo signals processed by the reception delaying unit 126.

The image processor 150 generates an ultrasound image by scan-converting ultrasound data generated by the ultrasound transceiver 115 and displays the ultrasound image.

An ultrasound image may be not only a grayscale ultrasound image obtained by scanning an object in an amplitude (A) mode, a brightness (B) mode, and a motion (M) mode, but also a blood flow Doppler image showing flow of blood (also referred to as a color Doppler image), a tissue Doppler image showing motion of tissues, and a spectral Doppler image showing moving speed of an object as a waveform.

A B mode processor 141 extracts B mode components from ultrasound data and processes the B mode components. An image generating unit 155 may generate an ultrasound image indicating signal intensities as brightness based on the extracted B mode components.

Similarly, a Doppler processor 142 may extract Doppler components from ultrasound data, and the image generating unit 155 may generate a Doppler image indicating motion of an object as colors or waveforms based on the extracted Doppler components.

The image generating unit 155 according to an embodiment of the present invention may generate a 2D ultrasound image via volume-rendering of volume data and may also generate an elasticity image which visualizes deformation of an object 10 due to pressure. Furthermore, the image generating unit 155 may display various pieces of additional information in an ultrasound image by using texts and graphics. The generated ultrasound image may be stored in the memory 180.

The shaking detecting unit 130 may detect shaking of the probe 20. When an ultrasound image is a 2D image, the shaking detecting unit 130 may detect a motion of at least one reference pixel included in the ultrasound image to detect the shaking of the probe 20. For example, the shaking detecting unit 130 may acquire as coordinates position information of a reference pixel included in a region of interest (ROI) in a plurality of frame images, and calculate a motion vector value of the reference pixel.

In this case, the reference pixel may be a pixel at a boundary of the ROI, a pixel which at the center of the ROI, or a pixel at a certain position. However, the present embodiment is not limited thereto. The shaking detecting unit 130 may select at least one pixel included in the ROI by using various methods, and set the selected pixel to the reference pixel.

When an ultrasound image is a 3D image, the shaking detecting unit 130 may detect a motion of a reference voxel included in an ROI to detect shaking of the probe 20.

Moreover, the shaking detecting unit 130 may include a sensor and detect shaking of the probe 20. Examples of the sensor may include an acceleration sensor, a gyro sensor, a proximity sensor. The acceleration sensor is an element that converts a one-direction acceleration change into an electrical signal. Such a sensor is widely used due to the advancements in micro-electromechanical system (MEMS) technology. Also, the gyro sensor is a sensor that measures an angular speed, and senses a direction twisted with respect to a reference direction.

The proximity sensor denotes a sensor that detects an object approaching a detection surface or an object near the detection surface by using an electromagnetic force or infrared light without any mechanical contact.

Examples of the proximity sensor include a transmissive photosensor, a directly reflective photosensor, a mirror reflective photosensor, a high frequency oscillation-type proximity sensor, a capacitive proximity sensor, a magnetic proximity sensor, and an infrared proximity sensor.

According to an embodiment of the present invention, the sensor may sense a moving speed of a probe, an angle at which the probe moves with respect to an object, a moving range of the probe, and whether the probe touches the object.

The display unit 160 displays the ultrasound image generated by the image generating unit 155. The display unit 160 may display various pieces of information processed by the ultrasound diagnostic apparatus 100, in addition to the ultrasound image, on a screen through a graphics user interface (GUI). The ultrasound diagnostic apparatus 100 may include two or more display units 160 depending on an implementation type.

The display unit 160 includes at least one of a liquid crystal display (LCD), a thin film transistor-liquid crystal display (TFT-LCD), an organic light-emitting diode (OLED), a flexible display, a 3D display, and an electrophoretic display.

Moreover, when the display unit 160 and the user input unit 190 are implemented as a touch screen having a layer structure, the display unit 160 may be used as an input unit that enables information to be input by a user's touch, in addition to an output unit.

The touch screen may be configured to detect a touch pressure in addition to a touch input position and a touched area. Also, the touch screen may be configured to detect a proximity touch as well as a real touch.

Herein, the term "real touch" denotes a case in which a pointer really touches a screen, and the term "proximity touch" denotes a case in which the pointer does not actually touch the screen but approaches the screen by a certain distance. A pointer used herein denotes a touch instrument for really touching or proximity-touching a specific portion of a displayed screen. Examples of the pointer include an electronic pen, a finger, etc.

Although not shown, the ultrasound diagnostic apparatus 100 may include various sensors inside or near the touch screen, for detecting a real touch or a proximity touch on the touch screen. An example of a sensor for sensing a touch of the touch screen is a tactile sensor.

The communicator 170 is connected to a network 30 in a wired or wireless manner to communicate with an external device or server. The communicator 170 may exchange data with a hospital server or a medical apparatus of a hospital which is connected thereto through a medical image information system (a PACS). Also, the communicator 170 may perform data communication according to the digital imaging and communications in medicine (DICOM) standard.

The communicator 170 may transmit and receive data, such as an ultrasound image, ultrasound data, Doppler data, etc. of an object, associated with a diagnosis of the object over the network 30, and may also transmit and receive a medical image captured by a medical apparatus such as a computed tomography (CT) apparatus, a magnetic resonance imaging (MRI) apparatus, or an X-ray apparatus. Furthermore, the communicator 170 may receive information on a diagnosis history or treatment schedule of a patient from a server, and use a diagnosis of an object. In addition, the communicator 170 may perform data communication with a portable terminal of a doctor or a patient, in addition to a server or medical apparatus of a hospital.

The communicator 170 may be connected to the network 30 in a wired or wireless manner, and may exchange data with a server 32, a medical apparatus 34, or a portable terminal 36. The communicator 170 may include one or more elements that enable communication with an external device, and for example, include a short-distance communication module 171, a wired communication module 172, and a mobile communication module 173.

The short-distance communication module 171 denotes a module for short-distance communication within a certain distance. Short-distance communication technology, according to an embodiment of the present invention, may include wireless LAN, Wi-Fi, Bluetooth, Zigbee, Wi-Fi direct (WFD), ultra wideband (UWB), infrared data association (IrDA), Bluetooth low energy (BLE), and near field communication (NFC), but the short-distance communication technology is not limited thereto.

The wired communication module 172 denotes a module for communication using an electrical signal or an optical signal. Wired communication technology according to an embodiment may include a pair cable, a coaxial cable, an optical fiber cable, or an Ethernet cable.

The mobile communication module 173 transmits and receives a radio frequency (RF) signal to and from a base station, an external terminal, and a server over a mobile communication network. The RF signal may include various types of data based on transmission and reception of a voice call signal, a video call signal, or a letter/multimedia message.

The memory 180 stores various pieces of information processed by the ultrasound diagnostic apparatus 100. For example, the memory 180 may store medical data, such as input/output ultrasound data and ultrasound images, associated with a diagnosis of an object, and may also store an algorithm or a program which is executed in the ultrasound diagnostic apparatus 100.

The memory 180 may be configured with various kinds of storage mediums such as a flash memory, a hard disk, an EEPROM, etc. Also, the ultrasound diagnostic apparatus 100 may operate a web storage or a cloud server, which performs a storage function of the memory 180 on a web.

The user input unit 190 generates input data which is input by a user for controlling an operation of the ultrasound diagnostic apparatus 100. The user input unit 190 may include hardware elements such as a keypad, a mouse, a touch pad, a trackball, a jog switch, but is not limited thereto. As another example, the user input unit 190 may further include various sensors such as an electrocardiogram (ECG) measurement module, a breath measurement sensor, a voice recognition sensor, a gesture recognition sensor, a fingerprint recognition sensor, an iris recognition sensor, a depth sensor, a distance sensor, etc.

In particular, the user input unit 190 may further include the touch screen in which the touch pad and the display unit 160 form the layer structure.

In this case, the ultrasound diagnostic apparatus 100 may display a specific mode ultrasound image and a control panel for an ultrasound image, on the touch screen. In addition, the ultrasound diagnostic apparatus 100 may sense a user's touch gesture for an ultrasound image through the touch screen.

The ultrasound diagnostic apparatus 100 according to an embodiment of the present invention may physically include some buttons, frequently used by a user, from among a plurality of buttons included in a control panel of general ultrasound diagnostic apparatuses, and the other buttons may be provided through a type of GUI on the touch screen.

The controller 195 controls an overall operation of the ultrasound diagnostic apparatus 100. That is, the controller 195 may control operations between the probe 20, the ultrasound transceiver 115, the image processor 150, the communicator 170, the memory 180, and the user input unit 190 which are illustrated in FIG. 1.

Some or all of the probe 20, the ultrasound transceiver 115, the image processor 150, the communicator 170, the memory 180, the user input unit 190, the shaking detector, and the controller 195 may be operated by a software module, but are not limited thereto. Some of the above-described elements may be operated by a hardware module. Also, at least some of the ultrasound transceiver 115, the shaking detector, the image processor 150, and the communicator 170 may be included in the controller 195, but are not limited to the implementation type.

The ultrasound diagnostic apparatus 100 according to an embodiment of the present invention may be implemented as a portable type as well as a card type. Examples of the portable diagnostic apparatuses may include picture archiving and communication system (PACS) viewers, smartphones, laptop computers, personal digital assistants (PDAs), tablet personal computers (PCs), etc., but are not limited thereto.

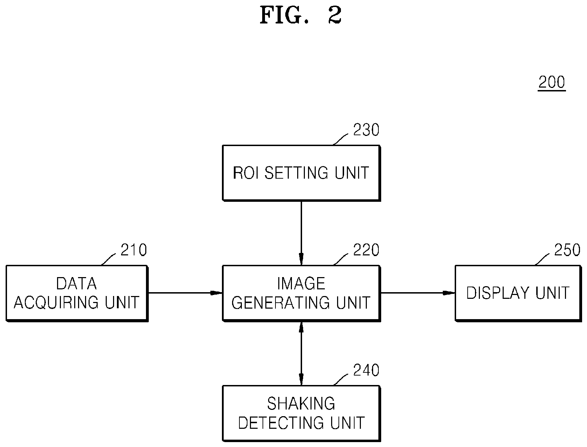

FIG. 2 is a block diagram illustrating a configuration of an ultrasound diagnostic apparatus 200 according to an embodiment of the present invention.

Referring to FIG. 2, the ultrasound diagnostic apparatus 200 may include a data acquiring unit 210, an image generating unit 220, an ROI setting unit 230, a shaking detecting unit 240, and a display unit 250.

The ROI setting unit 230 of FIG. 2 is an element corresponding to the user input unit 190 of FIG. 1, and the ROI setting unit 230 may set an ROI intended to be enlarged in an ultrasound image, based on a user input.

The ROI setting unit 230 may set an ROI. For example, by using a mouse or a touch screen, the ROI setting unit 230 may set the ROI on the basis of a user input which selects the ROI. Alternatively, the ROI setting unit 230 may set the ROI by using an eyeball mouse, a method of measuring an eyeball position and look direction of a user, or a probe. However, the present embodiment is not limited thereto, and the ROI setting unit 230 may set the ROI by using various methods.

The data acquiring unit 210 of FIG. 2 is an element corresponding to the probe 20 or ultrasound transceiver 115 of FIG. 1. The data acquiring unit 210 may transmit an ultrasound signal to an object, receive an echo signal reflected from the object, process the received echo signal, and acquire ultrasound data.

According to an embodiment of the present invention, the data acquiring unit 210 may acquire ultrasound data corresponding to a candidate region including the set ROI.

The image generating unit 220 of FIG. 2 is an element corresponding to the image generating unit 155 of FIG. 1, and may generate an ultrasound image based on the acquired ultrasound data. According to an embodiment of the present invention, the image generating unit 220 may generate a candidate image corresponding to the candidate region.

The shaking detecting unit 240 of FIG. 2 is an element corresponding to the shaking detecting unit 130 of FIG. 1. The shaking detecting unit 240 may detect a motion of at least one reference pixel or reference voxel included in the ultrasound image to detect shaking of a probe. Alternatively, the shaking detecting unit 240 may detect the shaking of the probe by using a sensor.

Moreover, the shaking detecting unit 130 may determine whether a sensed value, which is obtained by sensing a motion of the reference pixel or reference voxel or a motion of the probe, corresponds to a certain frequency band. For example, the certain frequency band may be about 5 Hz to about 15 Hz, and the shaking detecting unit 130 may filter the sensing value by using a band-pass filter of the certain frequency band to detect the shaking of the probe.

The display unit 250 of FIG. 2 is an element corresponding to the display unit 160 of FIG. 1. The display unit 250 may display a portion of a candidate image corresponding to the ROI among a plurality of the generated candidate images, based on the detected shaking of the probe.

For example, the display unit 250 may display the portion of the candidate image, corresponding to the ROI which is moved in the same direction as a motion vector of the reference pixel or reference voxel, from among the plurality of candidate images.

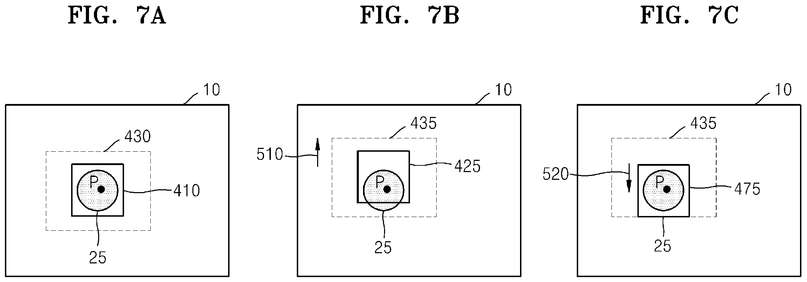

Alternatively, the display unit 250 may display the portion of the candidate image, corresponding to the ROI which is moved in a direction opposite to a shaking value of the probe, from among the plurality of candidate images. Therefore, the ultrasound diagnostic apparatus 200 may correct the shaking of the probe. This will be described below in detail with reference to FIG. 7.

The block diagram of each of the ultrasound diagnostic apparatuses 100 and 200 of FIGS. 1 and 2 is a block diagram according to an embodiment of the present invention. The elements of the block diagram may be integrated, added, or omitted depending on a specification of an actually implemented cache memory system. That is, depending on a specific case, two or more elements may be integrated into one element, or one element may be subdivided into two or more elements. Also, a function performed by each element is for describing an embodiment of the present invention, and each element or a detailed operation thereof does not limit the scope and spirit of the present invention.

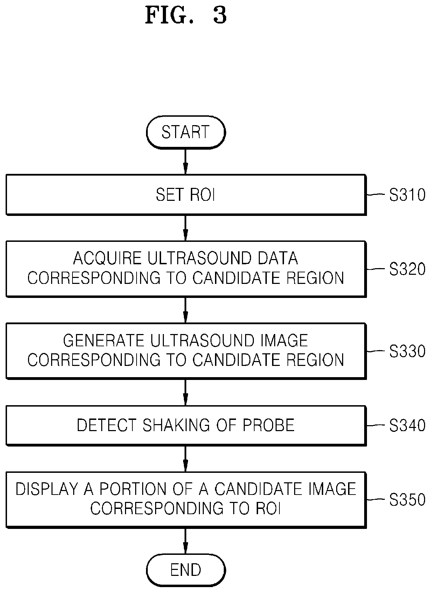

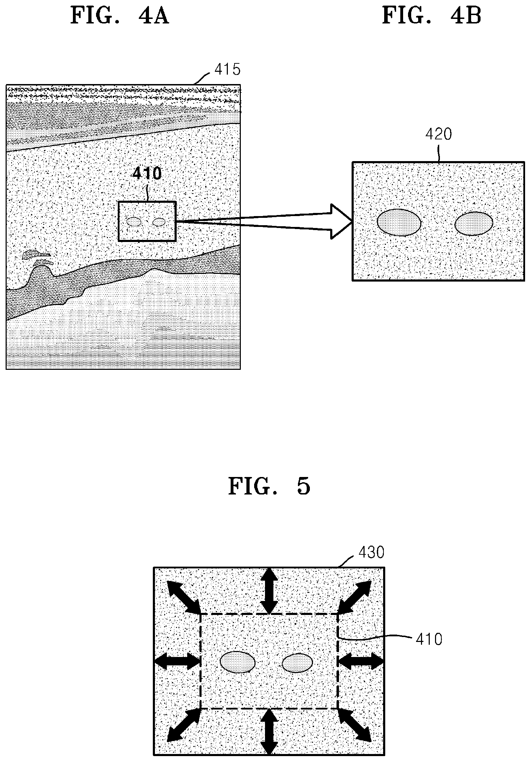

FIG. 3 is a flowchart illustrating a method of operating an ultrasound diagnostic apparatus, according to an embodiment of the present invention. FIGS. 4 to 8 are diagrams for describing an operating method of FIG. 3.

Referring to FIG. 3, in operation S310, the ultrasound diagnostic apparatus 100 (200) may set an ROI in an ultrasound image of an object. The ROI may be enlarged.

Referring to FIG. 4, for example, the ultrasound diagnostic apparatus 100 (200) may display an ultrasound image 415 of an object in the display unit 160 (250), and may set, an ROI 410 in the ultrasound image 415 displayed by the display unit 160 (250).

Therefore, as illustrated in FIG. 4 (b), the ultrasound diagnostic apparatus 100 (200) may display an enlarged image 420, in which an ultrasound image corresponding to a region set as an ROI is enlarged, on the display unit 160 (250).

Moreover, the ultrasound diagnostic apparatus 100 (200) may set an ROI in an ultrasound image based on a user input. For example, the ultrasound diagnostic apparatus 100 (200) may receive a user input for selecting the ROI 410 by using a mouse or a touch screen to set the ROI 410.

Alternatively, the ROI 410 may be set by using an eyeball mouse, a method of measuring an eyeball position or look direction of a user, or a probe. However, the present embodiment is not limited thereto. The ROI 410 may be set by various methods.

The ultrasound diagnostic apparatus 100 (200) may acquire ultrasound data corresponding to a candidate region including an ROI in operation S320, and generate an ultrasound image corresponding to the candidate region in operation S330.

For example, as illustrated in FIG. 5, a candidate region 430 may include the set ROI 410, and have a larger area than that of the ROI 410. Also, when the ultrasound image is a 3D ultrasound image, the candidate region 430 may include the set ROI 410 and have a larger volume than that of the ROI 410.

In this case, the area or volume of the candidate region 430 may increase or decrease in proportion to a magnification degree of the ROI 410. Alternatively, and in accordance with the present invention, the area or volume of the candidate region 430 may increase or decrease in proportion to a degree of shaking of the probe.

Moreover, as illustrated in FIG. 5, the candidate region 430 may be a region which is obtained by increasing the ROI 410 by the same amount in up, down, left, right, and diagonal directions. However, the present embodiment is not limited thereto. When the shaking of the probe is large in up and down directions, the candidate region 430 may be set so that an increase in up and down directions is larger than an increase in left and right directions. Alternatively, when the shaking of the probe is large in left and right directions, the candidate region 430 may be set so that the increase in left and right directions is larger than the increase in up and down directions. In addition, the ultrasound diagnostic apparatus 100 (200) may set the candidate region 430 by using various methods.

The ultrasound diagnostic apparatus 100 (200) may acquire ultrasound data corresponding to the candidate region 430 to generate an ultrasound image. In this case, the ultrasound image may be an ultrasound image obtained by enlarging an ultrasound image by a predetermined magnification degree.

In operation S340, the ultrasound diagnostic apparatus 100 (200) may detect the shaking of the probe.

The ultrasound diagnostic apparatus 100 (200) may detect a motion of at least one reference pixel included in the ROI 410 to detect the shaking of the probe.

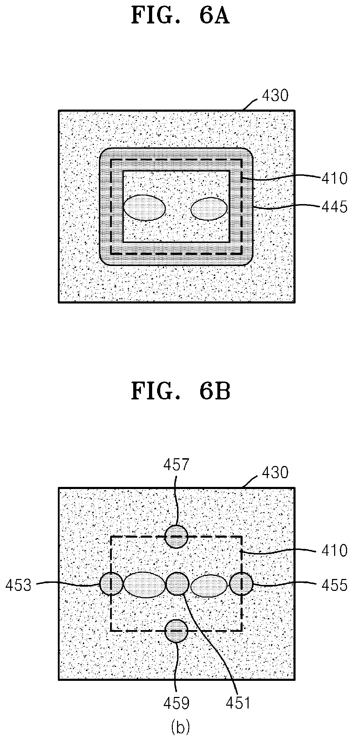

For example, as illustrated in FIG. 6 (a), the reference pixel may be a pixel at a boundary region 445 of the ROI 410 in the ultrasound image. Alternatively, as illustrated in FIG. 6 (b), the reference pixel may be a pixel in a central region 451 of the ROI 410, or may be a pixel at each of up, down, left, and right boundary regions 457, 459, 453 and 455 with respect to the central region 451 of the ROI 410. However, the present embodiment is not limited thereto, and the reference pixel may be a pixel selected by various methods.

When the ultrasound image is a 3D image, the ultrasound diagnostic apparatus 100 (200) may detect a motion of at least one reference voxel included in the ROI 410 to detect the shaking of the probe, and the reference voxel may be selected by various methods.

Moreover, the ultrasound diagnostic apparatus 100 (200) may analyze a plurality of ultrasound image frames, calculate a motion vector of the reference pixel, and detect the shaking of the probe.

For example, as illustrated in FIG. 7, the ultrasound diagnostic apparatus 100 (200) may set a reference pixel P in an object of interest (OOI), and calculate a motion vector of the reference pixel P, based on coordinates (first coordinates and relative coordinates of the first ROI 410) of the reference pixel P before the probe moves and coordinates (second coordinates and relative coordinates of a second ROI 425) of the reference pixel P after the probe moves, thereby detecting the shaking of the probe.

When the ultrasound image is a 3D image, the ultrasound diagnostic apparatus 100 (200) may calculate a motion vector of a reference voxel by using the same method as the above-described method.

Alternatively, the ultrasound diagnostic apparatus 100 (200) may detect the shaking of the probe by using a sensor. For example, the ultrasound diagnostic apparatus 100 (200) may include sensors such as an acceleration sensor, a gyro sensor, a proximity sensor, a tactile sensor, and a temperature sensor. The ultrasound diagnostic apparatus 100 (200) may sense a moving speed of a probe, an angle at which the probe moves with respect to an object, and a moving range of the probe. Therefore, the ultrasound diagnostic apparatus 100 (200) may calculate a motion 510 of the probe based on the sensed value.

Moreover, the ultrasound diagnostic apparatus 100 (200) may detect the shaking of the probe by using a combination of a detection method based on a reference pixel and a detection method based on sensing.

The ultrasound diagnostic apparatus 100 (200) may determine whether a motion of the probe calculated based on the reference pixel and a motion of the probe calculated based on a value obtained by sensing the motion of the probe correspond to a certain frequency band, thereby detecting the shaking of the probe corresponding to the certain frequency band. For example, the ultrasound diagnostic apparatus 100 (200) may detect the shaking of the probe corresponding to the certain frequency band by using a band-pass filter of the certain frequency band. In this case, the certain frequency band may be a frequency band which is generated by shaking of a hand, and may be about 5 Hz to about 15 Hz.

Therefore, the ultrasound diagnostic apparatus 100 (200) may detect only a motion of the probe corresponding to the certain frequency band from among calculated motions of the probe, and may determine a motion of the probe which does not correspond to the certain frequency band from among the calculated motions of the probe as a motion when the probe is intentionally moved by the user.

In operation S350, the ultrasound diagnostic apparatus 100 (200) may display a portion of a candidate image corresponding to the ROI 410 based on the detected shaking of the probe. This will be described in detail with reference to FIG. 7.

Referring to FIG. 7, as illustrated in FIG. 7 (a), when it is desired to acquire an ultrasound image of an OOI 25 included in an object 10, a user may dispose a probe in an OOI region (an ROI) 410. In this case, the probe may acquire an ultrasound image (a first candidate image) corresponding to a first candidate region 430 including the ROI 410, and an ultrasound image corresponding to the ROI 410 from among a plurality of the first candidate images may be enlarged and displayed on a display unit.

When the probe is shaken by shaking of the user's hand, for example, when the probe moves (510) in an up direction with respect to the object 10, as illustrated in FIG. 7 (b), the probe may acquire a second candidate image corresponding to a second candidate region 435. In this case, when an ultrasound image corresponding to an ROI 425 from among a plurality of ultrasound images corresponding to the acquired second candidate region is displayed without correcting the shaking of the hand, the OOI 25 is shown as being moved in a downward direction in a displayed image.

Therefore, in order to correct the shaking of the probe, as illustrated in FIG. 7 (c), the ultrasound diagnostic apparatus 100 (200) may move (520) the ROI 425 in a direction opposite the motion 510 of the probe in an ultrasound image corresponding to the acquired second candidate region 435, and display an ultrasound image corresponding to a moved ROI 475 on the display unit.

As described above, the ultrasound diagnostic apparatus 100 (200) may move the ROI 425 in a direction opposite the shaking of the probe, and display the ultrasound image corresponding to the moved ROI 475, thereby providing an ultrasound image in which the shaking of the probe is corrected. In this case, in order to calculate the shaking of the probe, as described above, the ultrasound diagnostic apparatus 100 (200) may sense a shaking value of the probe, or calculate a motion vector of a reference pixel P.

For example, the ultrasound diagnostic apparatus 100 (200) may sense a moving value 510 of the probe, and move the ROI 425 by a sensed motion amount of the probe in a direction opposite a moving direction of the probe.

Alternatively, the ultrasound diagnostic apparatus 100 (200) may set the reference pixel P in the OOI 25, and calculate a motion vector of the reference pixel P, based on coordinates (relative coordinates of a first ROI 410) of the reference pixel P before the probe moves and coordinates (relative coordinates of a second ROI 425) of the reference pixel P after the probe moves.

The ultrasound diagnostic apparatus 100 (200) may move the ROI in the same direction and by the same amount as the motion vector so that the coordinates (the relative coordinates of the second ROI 425) of the reference pixel P after the probe moves are the same as the coordinates (the relative coordinates of the first ROI 410) of the reference pixel P before the probe moves. Alternatively, the ultrasound diagnostic apparatus 100 (200) may display an ultrasound image so that the reference pixel P of the OOI 25 is disposed at the coordinates (the relative coordinates of the first ROI 410) of the reference pixel P before the probe moves.

Therefore, as illustrated in FIG. 7 (c), even when the probe is shaken by the shaking of the hand, the OOI 25 may be displayed at the same position without being shaken in a displayed ultrasound image.

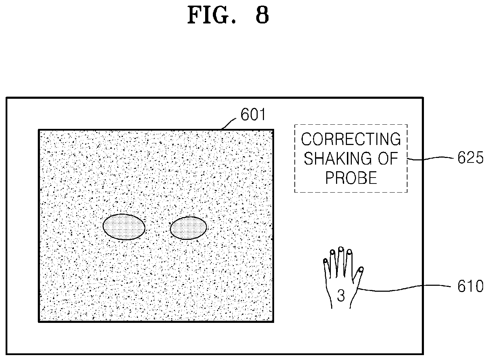

The ultrasound diagnostic apparatus 100 (200) may display a degree of shaking of the probe in the display unit 160 (250). For example, the ultrasound diagnostic apparatus 100 (200) may display a first region 601 of the display unit 160 (250), and as illustrated in FIG. 8, may display an icon 610 representing the degree of shaking of the probe in a second region.

The ultrasound diagnostic apparatus 100 (200) may display an icon to be shaken in left and right directions or up and down directions depending on the degree of shaking of the probe, and may display the degree of shaking of the probe as a numerical value. In addition, the ultrasound diagnostic apparatus 100 (200) may display a message indicating that the degree of shaking of the probe is large, and also may display a message 625 indicating that an ultrasound image in which the shaking of the probe is corrected is displayed.

As described above according to the one or more of the above embodiments, shaking of an ultrasound image is corrected when the ultrasound image is enlarged.

Moreover, an ultrasound image in which undesired shaking is corrected is provided, thereby increasing a degree of accuracy of a diagnosis.

The ultrasound diagnostic apparatus and the method of operating the same according to embodiments of the present invention may also be embodied as computer readable codes on a computer readable recording medium. The computer readable recording medium is any data storage device that can store data which can be thereafter read by a computer system. Examples of the computer readable recording medium include read-only memory (ROM), random-access memory (RAM), CD-ROMs, magnetic tapes, floppy disks, and optical data storage. The computer readable recording medium can also be distributed over network coupled computer systems so that the computer readable code may be stored and executed in a distributed fashion.

It should be understood that the exemplary embodiments described herein should be considered in a descriptive sense only and not for purposes of limitation.

While one or more embodiments of the present invention have been described with reference to the figures, it will be understood by those of ordinary skill in the art that various changes in form and details may be made therein without departing from the scope of the present invention as defined by the following claims.

## Claims

1. A method of operating an ultrasound diagnostic apparatus (100,200), the method comprising:
setting a region of interest (ROI) (410) intended to be enlarged in an ultrasound image of an object (10);
acquiring ultrasound data corresponding to a candidate region (430) including the ROI (410) using a probe (20);
generating a candidate image corresponding to the candidate region (430), based on the acquired ultrasound data;
detecting shaking of the probe (20) by means of a shaking detecting unit (130, 240); and
displaying by means of a display unit (160, 250) a portion of the candidate image corresponding to the ROI (410), based on the detected shaking of the probe (20),
wherein the candidate image is obtained by enlarging an ultrasound image corresponding to the candidate region (430), and
the area of the candidate region (430) is proportional to a degree of shaking of the probe (20).

2. The method of claim 1, wherein the detecting of the shaking comprises detecting a motion of at least one reference pixel included in the ROI (410) from among a plurality of pixels included in the candidate image.

3. The method of claim 2, wherein,
the detecting of the shaking comprises calculating a motion vector indicating the motion of the at least one reference pixel, and
the displaying of the portion of the one candidate image comprises moving the set ROI (410) in the same direction as a direction of the motion vector, and displaying the portion of the candidate image corresponding to the moved ROI (410) from among the plurality of candidate images.

4. The method of claim 1, wherein the detecting of the shaking comprises calculating a motion of the probe (20) to determine whether the motion of the probe (20) corresponds to a certain frequency band.

5. The method of claim 1, wherein the detecting of the shaking comprises sensing the shaking of the probe (20) by using a sensor.

6. The method of claim 5, wherein the displaying of the portion of the candidate image comprises moving the set ROI (410) in a direction opposite to a sensed value to display the portion of the candidate image corresponding to the moved ROI (410) from among the plurality of candidate images.

7. The method of claim 1, further comprising displaying the detected shaking of the probe (20).

8. An ultrasound diagnostic apparatus (100,200) comprising:
a ROI setting unit (230) adapted to set a region of interest (ROI) (410) intended to be enlarged in an ultrasound image of an object (10);
a data acquiring unit (210) adapted to acquire ultrasound data corresponding to a candidate region (430) including the ROI (410) using a probe (20);
an image generating unit (155,220) adapted to generate a candidate image corresponding to the candidate region (430), based on the acquired ultrasound data;
a shaking detecting unit (130,240) adapted to detect shaking of the probe (20); and
a display unit (160,250) adapted to display a portion of the candidate image corresponding to the ROI (410), based on the detected shaking of the probe (20),
wherein the candidate image is obtained by enlarging an ultrasound image corresponding to the candidate region (430), and
the area of the candidate region (430) is proportional to a degree of shaking of the probe (20).

9. The ultrasound diagnostic apparatus (100,200) of claim 8, wherein the shaking detecting unit (130,240) is adapted to detect a motion of at least one reference pixel included in the ROI (410) from among a plurality of pixels included in the candidate image.

10. The ultrasound diagnostic apparatus (100,200) of claim 9, wherein, the shaking detecting unit (130,240) is adapted to calculate a motion vector indicating the motion of the at least one reference pixel, and the display unit (160,250) is adapted to move the set ROI (410) in the same direction as a direction of the motion vector, and to display the portion of the candidate image corresponding to the moved ROI (410) from among the plurality of candidate images.

11. The ultrasound diagnostic apparatus (100,200) of claim 8, wherein the shaking detecting unit (130,240) is adapted to calculate a motion of the probe (20) to determine whether the motion of the probe (20) corresponds to a certain frequency band.

12. The ultrasound diagnostic apparatus (100,200) of claim 8, wherein the shaking detecting unit (130,240) is adapted to sense the shaking of the probe (20) by using a sensor.

13. The ultrasound diagnostic apparatus (100,200) of claim 12, wherein the display unit (160,250) is adapted to move the set ROI (410) in a direction opposite to a sensed value to display the portion of the candidate image corresponding to the moved ROI (410) from among the plurality of candidate images.

## Patentansprüche

1. Verfahren zum Betreiben einer Ultraschalldiagnosevorrichtung (100,200), wobei das Verfahren Folgendes aufweist:
Festlegen eines interessierenden Bereichs (ROI) (410), der dafür vorgesehen ist, in einem Ultraschallbild eines Objekts (10) vergrößert zu werden;
Erlangen von Ultraschalldaten, die mit einem den ROI (410) einschließenden Kandidatenbereich (430) korrespondieren, unter Verwendung einer Sonde (20);
Erzeugen eines Kandidatenbilds, das mit dem Kandidatenbereich (430) korrespondiert, basierend auf den erlangten Ultraschalldaten;
Detektieren eines Wackelns der Sonde (20) mittels einer Wackeldetektiereinrichtung (130,240); und
Anzeigen eines Bereichs des Kandidatenbilds, das mit dem ROI (410) korrespondiert, mittels einer Anzeigeeinheit (160,250), basierend auf dem ermittelten Wackeln der Sonde (20),
wobei das Kandidatenbild durch Vergrößern eines Ultraschallbilds erlangt wird, das mit dem Kandidatenbereich (430) korrespondiert, und
wobei die Fläche des Kandidatenbereichs (430) proportional zu einem Grad des Wackelns der Sonde (20) ist.

2. Verfahren nach Anspruch 1, wobei das Detektieren des Wackelns das Detektieren einer Bewegung wenigstens eines in dem ROI (410) enthaltenen Referenzpixels unter einer Vielzahl von in dem Kandidatenbild enthaltenen Pixeln aufweist.

3. Verfahren nach Anspruch 2, wobei das Detektieren des Wackelns das Berechnen eines Bewegungsvektors aufweist, der die Bewegung des wenigstens einen Referenzpixels anzeigt, und
wobei das Anzeigen des Bereichs des einen Kandidatenbilds das Bewegen des festgelegten ROI (410) in derselben Richtung wie eine Richtung des Bewegungsvektors und das Anzeigen des Bereichs des Kandidatenbilds, das mit dem bewegten ROI (410) korrespondiert, unter der Vielzahl von Kandidatenbildern aufweist.

4. Verfahren nach Anspruch 1, wobei das Detektieren des Wackelns das Berechnen einer Bewegung der Sonde (20) aufweist, um zu ermitteln, ob die Bewegung der Sonde (20) mit einem bestimmen Frequenzband korrespondiert.

5. Verfahren nach Anspruch 1, wobei das Detektieren des Wackelns das Erkennen des Wackelns der Sonde (20) unter Verwendung eines Sensors aufweist.

6. Verfahren nach Anspruch 5, wobei das Anzeigen des Bereichs des Kandidatenbilds das Bewegen des festgelegten ROI (410) in einer Richtung entgegengesetzt zu einem ermittelten Wert aufweist, um den Bereich des Kandidatenbilds, der mit dem bewegten ROI (410) korrespondiert, unter der Vielzahl von Kandidatenbildern anzuzeigen.

7. Verfahren nach Anspruch 1, welches des Weiteren das Anzeigen des detektierten Wackelns der Sonde (20) aufweist.

8. Ultraschalldiagnosevorrichtung (100,200), welche Folgendes aufweist:
eine ROI-Festlegeeinheit (230), die dafür vorgesehen ist, einen interessierenden Bereich (ROI) (410) festzulegen, der dafür vorgesehen ist, in einem Ultraschallbild eines Objekts (10) vergrößert zu werden;
eine Datenerlangungseinheit (210), die dafür vorgesehen ist, Ultraschalldaten, die mit einem den ROI (410) beinhaltenden Kandidatenbereich (430) korrespondieren, unter Verwendung einer Sonde (20) zu erlangen;
eine Bilderzeugungseinheit (155,220), die dafür vorgesehen ist, ein Kandidatenbild, das mit dem Kandidatenbereich (430) korrespondiert, basierend auf den erlangten Ultraschalldaten zu erzeugen;
eine Wackeldetektiereinheit (130,240), die dafür vorgesehen ist, ein Wackeln der Sonde (20) zu detektieren; und
eine Anzeigeeinheit (160,250), die dafür vorgesehen ist, einen Bereich des Kandidatenbilds, das mit dem ROI (410) korrespondiert, basierend auf dem detektierten Wackeln der Sonde (20) anzuzeigen,
wobei das Kandidatenbild durch Vergrößern eines Ultraschallbilds erlangt wird, das mit dem Kandidatenbereich (430) korrespondiert, und
wobei die Fläche des Kandidatenbereichs (430) proportional zu einem Grad des Wackelns der Sonde (20) ist.

9. Ultraschalldiagnosevorrichtung (100,200) nach Anspruch 8, wobei die Wackeldetektiereinheit (130,240) dafür vorgesehen ist, eine Bewegung wenigstens eines in dem ROI (410) enthaltenen Referenzpixels unter einer Vielzahl von in dem Kandidatenbild enthaltenen Pixeln zu detektieren.

10. Ultraschalldiagnosevorrichtung (100,200) nach Anspruch 9, wobei die Wackeldetektiereinheit (130,240) dafür vorgesehen ist, einen Bewegungsvektor, der die Bewegung des wenigstens einen Referenzpixels anzeigt, zu berechnen, und
wobei die Anzeigeeinheit (160,250) dafür vorgesehen ist, den festgelegten ROI (410) in derselben Richtung wie eine Richtung des Bewegungsvektors zu bewegen und den Bereich des Kandidatenbilds, das mit dem bewegten ROI (410) korrespondiert, unter der Vielzahl von Kandidatenbildern anzuzeigen.

11. Ultraschalldiagnosevorrichtung (100,200) nach Anspruch 8, wobei die Wackeldetektiereinheit (130,240) dafür vorgesehen ist, eine Bewegung der Sonde (20) zu berechnen, um zu ermitteln, ob die Bewegung der Sonde (20) mit einem bestimmten Frequenzband korrespondiert.

12. Ultraschalldiagnosevorrichtung (100,200) nach Anspruch 8, wobei die Wackeldetektiereinheit (130,240) dafür vorgesehen ist, das Wackeln der Probe (20) durch Verwenden eines Sensors zu erkennen.

13. Ultraschalldiagnosevorrichtung (100,200) nach Anspruch 12, wobei die Anzeigeeinheit (160,250) dafür vorgesehen ist, den festgelegten ROI (410) in einer Richtung entgegengesetzt zu einem ermittelten Wert zu bewegen, um den Bereich des Kandidatenbilds, der mit dem bewegten ROI (410) korrespondiert, unter der Vielzahl von Kandidatenbildern anzuzeigen.

## Revendications

1. Procédé de fonctionnement d'un appareil de diagnostic par ultrasons (100, 200), le procédé comprenant :
la définition d'une région d'intérêt (ROI) (410) destinée à être agrandie dans une image ultrasonore d'un objet (10) ;
l'acquisition de données ultrasonores correspondant à une région candidate (430) incluant la ROI (410) en utilisant une sonde (20) ;
la génération d'une image candidate correspondant à la région candidate (430), sur la base des données ultrasonores acquises ;
la détection de secousse de la sonde (20) au moyen d'une unité de détection de secousse (130, 240) ; et
l'affichage au moyen d'une unité d'affichage (160, 250) d'une partie de l'image candidate correspondant à la ROI (410), sur la base de la secousse détectée de la sonde (20),
dans lequel l'image candidate est obtenue en agrandissant une image ultrasonore correspondant à la région candidate (430), et
la surface de la région candidate (430) est proportionnelle à un degré de secousse de la sonde (20).

2. Procédé selon la revendication 1, dans lequel la détection de secousse comprend la détection d'un mouvement d'au moins un pixel de référence inclus dans la ROI (410) parmi une pluralité de pixels inclus dans l'image candidate.

3. Procédé selon la revendication 2, dans lequel la détection de secousse comprend le calcul d'un vecteur de mouvement indiquant le mouvement dudit au moins un pixel de référence, et
l'affichage de la partie de l'image candidate comprend le déplacement de la ROI définie (410) dans la même direction qu'une direction du vecteur de mouvement, et l'affichage de la partie de l'image candidate correspondant à la ROI déplacée (410) parmi la pluralité des images candidates.

4. Procédé selon la revendication 1, dans lequel la détection de secousse comprend le calcul d'un mouvement de la sonde (20) pour déterminer si le mouvement de la sonde (20) correspond à une certaine bande de fréquence.

5. Procédé selon la revendication 1, dans lequel la détection de secousse comprend la détection de secousse de la sonde (20) en utilisant un capteur.

6. Procédé selon la revendication 5, dans lequel l'affichage de la partie de l'image candidate comprend le déplacement de la ROI définie (410) dans une direction opposée à une valeur détectée pour afficher la partie de l'image candidate correspondant à la ROI déplacée (410) parmi la pluralité d'images candidates.

7. Procédé selon la revendication 1, comprenant en outre l'affichage de la secousse détectée de la sonde (20).

8. Appareil de diagnostic par ultrasons (100, 200) comprenant :
une unité de définition de ROI (230) adaptée pour régler une région d'intérêt (ROI) (410) destinée à être agrandie dans une image ultrasonore d'un objet (10) ;
une unité d'acquisition de données (210) adaptée pour acquérir des données ultrasonores correspondant à une région candidate (430) incluant la ROI (410) en utilisant une sonde (20) ;
une unité de génération d'image (155, 220) adaptée pour générer une image candidate correspondant à la région candidate (430), sur la base des données ultrasonores acquises ;
une unité de détection de secousse (130, 240) adaptée pour détecter une secousse de la sonde (20) ; et
une unité d'affichage (160, 250) adaptée pour afficher une partie de l'image candidate correspondant à la ROI (410), sur la base de la secousse détectée de la sonde (20),
dans lequel l'image candidate est obtenue en agrandissant une image ultrasonore correspondant à la région candidate (430), et
la surface de la région candidate (430) est proportionnelle à un degré de secousse de la sonde (20).

9. Appareil de diagnostic par ultrasons (100, 200) selon la revendication 8, dans lequel
l'unité de détection de secousse (130, 240) est adaptée pour détecter un mouvement d'au moins un pixel de référence inclus dans la ROI (410) parmi une pluralité de pixels inclus dans l'image candidate.

10. Appareil de diagnostic par ultrasons (100, 200) selon la revendication 9, dans lequel
l'unité de détection de secousse (130, 240) est adaptée pour calculer un vecteur de mouvement indiquant le mouvement dudit au moins un pixel de référence, et
l'unité d'affichage (160, 250) est adaptée pour déplacer la ROI définie (410) dans la même direction qu'une direction du vecteur de mouvement, et pour afficher la partie de l'image candidate correspondant à la ROI déplacée (410) parmi la pluralité des images candidates.

11. Appareil de diagnostic par ultrasons (100, 200) selon la revendication 8, dans lequel
l'unité de détection de secousse (130, 240) est adaptée pour calculer un mouvement de la sonde (20) pour déterminer si le mouvement de la sonde (20) correspond à une certaine bande de fréquence.

12. Appareil de diagnostic par ultrasons (100, 200) selon la revendication 8, dans lequel
l'unité de détection de secousse (130, 240) est adaptée pour détecter la secousse de la sonde (20) en utilisant un capteur.

13. Appareil de diagnostic par ultrasons (100, 200) selon la revendication 12, dans lequel
l'unité d'affichage (160, 250) est adaptée pour déplacer
la ROI définie (410) dans une direction opposée à une valeur détectée pour afficher la partie de l'image candidate correspondant à la ROI déplacée (410) parmi la pluralité des images candidates.
